# EUROPEAN PATENT APPLICATION

(11) **EP 4 592 681 A1**
(43) Date of publication of application: **30.07.2025**
(21) Application number: 24154318.0
(22) Date of filing: 29.01.2024
(51) Int. Cl.: G01N 33/68

(54) **ALLERGY TEST**

(71) Applicant: HVD Biotech Vertriebsgesellschaft m.b.H., 1150 Wien (AT)
(72) Inventor: Valenta, Rudolf, 1090 Wien (AT); Vrtala, Susanne, 1090 Wien (AT); Trifonova, Daria, 1090 Wien (AT); Dsouza, Nishelle, 1090 Wien (AT); Sarzsinszky, Eszter, 1090 Wien (AT); Gattinger, Pia, 1090 Wien (AT); Huan, Huey-Jy, 3500 Krems (AT)
(74) Representative: Schwarz & Partner Patentanwälte GmbH

(57) **Abstract**

The present invention relates to a method for determining the presence of IgE molecules binding to one or more allergens of non-mammalian origin in a sample comprising the step of contacting said sample with at least one allergen of non-mammalian origin or a functional fragment thereof, wherein the at least one allergen or the functional fragment has a mammalian or mammalian-like glycosylation pattern.

## Description

### TECHNICAL FIELD

The present invention relates to the field of allergy diagnosis, in particular of diagnosis of IgE-mediated allergies.

### BACKGROUND ART

Cross-reactive carbohydrate determinants (CCDs) (Altmann F. Int Arch Allergy Immunol 142(2007):99-115) are carbohydrate structures found in a variety of allergen sources, including grass, tree and weed pollen, arthropods, molds/fungi such as *Alternaria alternate, Cladosporium, Aspergillus,* yeast and *Malassezia,* plant derived food, e.g. fruits, vegetables, nuts and insect venom. The most common CCDs are alpha-1,3-fucose and beta-1,2 xylose, which are not found in mammalian cells and could lead to false positive results when using serological allergy diagnostic tests.

There are strategies that can be used to diminish false-positive results caused by CCD-specific IgE, including the use of CCD blockers or the mutation of N-glycosylation sites using recombinant allergens. However, there are some shortcomings: (i) in serum samples with high-levels of CCD-specific IgE, complete inhibition cannot be achieved; (ii) it has been shown, that plant-derived carbohydrate epitopes have a diversity, and thus cannot be blocked with one synthetic CCD inhibitor; (iii) removing N-glycosylation sites by amino acid mutation can negatively influence the fold, structure, function or allergenic activity of the allergen. In fact, the major bee venom allergen Api m 1 can be expressed without its N-glycosylation site in Sf9 insect cells, but with reduced allergenic activity (Gattinger, P et al. (2021). J Allergy Clin Immunol, 147(4), 1502-1504.e5. https://doi.org/10.1016/j.jaci.2020.10.002).

It is an object of the present invention to provide methods and means to bypass CCDs and the subsequent challenges during allergy diagnosis.

### SUMMARY OF THE INVENTION

Therefore, the present invention relates to a method for determining the presence of IgE molecules binding to one or more allergens of non-mammalian origin in a sample comprising the step of contacting said sample with at least one allergen of non-mammalian origin or a functional fragment thereof, wherein the at least one allergen or the functional fragment has a mammalian or mammalian-like glycosylation pattern.

CCD are sugar structures that are bound to proteins in the course of a biochemical reaction - the result are so-called glycoproteins. The CCD structures of glycosylated proteins from plants and invertebrates, such as insects, differ from those of mammalian, in particular human, glycoproteins and are therefore immunogenic. If the immune system has formed IgE antibodies after contact with an allergen, these can be directed against the protein part itself, against the CCD structure (anti-CCD IgE antibodies) or, in certain cases, against both. Anti-CCD IgE antibodies occur in up to 25% of allergic patients, but usually show no clinical relevance - in contrast to IgE antibodies against the specific protein regions (Altmann F. Int Arch Allergy Immunol 142(2007):99-115). In addition, anti-CCD IgE are highly cross-reactive and recognize the sugar residues of glycoproteins in diverse allergen sources such as plants, insects, crustaceans, mollusks or latex.

In allergy diagnostics based on measurement of IgE specific for allergen extracts or allergens purified from the allergen sources, anti-CCD IgE antibodies now pose a problem, because in the case of a positive test result it is not possible to distinguish whether this is based on the reaction of specific IgE antibodies against the protein, cross-reactive anti-CCD IgE antibodies or both antibody types. Hence, current serological allergy diagnostic tests lead in many cases to false positive results when using allergens of natural sources.

In order to overcome this problem, the method of the present invention uses allergens of non-mammalian origin which have a glycosylation pattern which does not react with human IgE antibodies. This can be achieved by the expression of allergens in mammalian cells (e.g., HEK or CHO cells) or non-mammalian cells having a mammalian or mammalian-like glycosylation pattern. Alternatively, a non-IgE-reactive glycosylation pattern may be achieved by engineering of non-mammalian eukaryotic cells to avoid the production of IgE-reactive CCDs (e.g., by silencing or genetic elimination of proteins conveying such a IgE-reactive glycosylation or by changing of proteins to resemble a machinery conveying a mammalian glycosylation). The person skilled in the art may consider modifying eukaryotic cells such as insect cells, yeast or plant cells to produce glycosylated allergens with mammalian-type glycosylation and thus avoid IgE-reactive glycosylation. Mammalian type glycosylation may be also achieved in engineered plant cells or other eukaryotic cells which are not mammalian or human cells (see e.g., EP 1 228 231).

It is known in the art, that mammalian cells (including HEK and CHO cells), in particular primate cells like human cells, are not able to attach alpha-1,3-fucose and beta-1,2-xylose moieties to their terminal N-glycan branches, which are known to be responsible for CCDs. Mammalian cells lack the respective enzymes for these reactions. Hence, the glycosylation pattern of the allergens used in the method of the present invention is clearly distinct from those of a natural source and different from recombinant allergens produced in insect cells, plant cells, yeast etc. which add alpha-1,3-fucose and beta-1,2-xylose moieties and thus produce CCDs (Altmann F. Int Arch Allergy Immunol 142 (2007) :99-115) .

Another aspect of the present invention relates to method for determining whether a sample comprises allergen specific IgE molecules binding to a cross-reactive carbohydrate determinant (CCD) of an allergen of non-mammalian origin or to the proteinaceous part of said allergen, comprising the steps of:
a) providing at least one first allergen of non-mammalian origin or at least one functional fragment thereof, wherein said at least one first allergen of non-mammalian origin or said at least one fragment thereof has a mammalian or mammalian-like glycosylation pattern, which does not react with human CCD-specific IgE antibodies,
b) providing at least one second allergen of non-mammalian origin or at least one functional fragment thereof comprising an amino acid sequence substantially identical to the at least one first allergen of non-mammalian origin or functional fragment thereof, wherein said at least one second allergen of non-mammalian origin or said at least one fragment thereof has a cross-reactive carbohydrate determinant,
c) contacting a first part of said sample with the at least one first allergen of non-mammalian origin or functional fragment thereof and a second part of said sample with the at least one second allergen of non-mammalian origin or functional fragment thereof.

As discussed above IgE molecules of patients sensitized to CCD comprising allergens may have a binding affinity to CCD. Since CCDs may also be present on other allergens, a binding test with naturally occurring allergens or allergens comprising CCDs may result in wrong-positive diagnoses. Hence, a test method for determining whether a sample comprises allergen specific IgE molecules binding to a CCD of an allergen or to the proteinaceous part of said allergen is helpful to avoid wrong-positive diagnoses and to set-up an allergen-specific therapy.

The method of the present invention cannot only be used for simultaneous testing (e.g., both allergens are on the same solid phase or on different solid phases and tested at the same time) but also for testing the binding of IgEs to allergens of non-mammalian origin comprising mammalian or mammalian-like glycosylation with corresponding CCD-bearing allergens and/or other allergens obtained at different time points and/or in different test runs. For example, serum is obtained at one time point and tested with the allergen comprising mammalian or mammalian-like glycosylation and serum obtained at the same or another time point may be tested for other allergen molecules at other time points. This allows to obtain a meaningful complete molecular sensitization profile comprising the testing with the allergen of non-mammalian origin comprising mammalian or mammalian-like glycosylation and testing with other allergen molecules may require testing at different time points.

Another aspect of the present invention relates to a method for determining a sensitization profile of an individual comprising the steps of:
a) providing a sample of an individual comprising IgE molecules and
b) contacting said sample with a multiplicity of allergens of non-mammalian origin or functional fragments thereof to determine whether IgEs comprised in the sample bind to one or more of said allergens or fragments thereof giving the sensitization profile of said individual, wherein
at least a part of said allergens of non-mammalian origin or functional fragments has a mammalian or mammalian-like glycosylation pattern.

A sensitization profile is a comprehensive assessment or analysis of an individual's sensitivities or reactions to allergens that trigger an allergic or hypersensitive response in an individual. This profile typically includes information about the severity of the reactions and the frequency of exposure. A sensitization profile helps to better understand an individual's sensitivities and develop appropriate management strategies or interventions. Furthermore, it allows also to monitor the progress of therapeutic measures.

A further aspect of the present invention relates to a method for diagnosing an allergy in a subject comprising the steps of:
a) providing at least one first allergen of non-mammalian origin or a functional fragment thereof, wherein said at least one first allergen of non-mammalian origin or said fragment thereof has a mammalian or mammalian-like glycosylation pattern,
b) providing at least one second allergen of non-mammalian origin or a functional fragment thereof comprising an amino acid sequence substantially identical to the at least one first allergen of non-mammalian origin or functional fragment thereof, wherein said at least one second allergen of non-mammalian origin or said fragment thereof has a glycosylation pattern naturally occurring on said at least one second allergen of non-mammalian origin,
c) contacting a first part of said sample with the at least one first allergen of non-mammalian origin or functional fragment thereof and a second part of said sample with the at least one second allergen of non-mammalian origin or functional fragment thereof,
d) diagnosing an allergy if binding of IgE molecules in the sample to the at least one first allergen of non-mammalian origin or functional fragment thereof and a reduced binding of IgE molecules in the sample to the at least one second allergen of non-mammalian origin or functional fragment thereof is determined..

A further aspect of the present invention relates to a diagnostic device for screening patients sensitized to at least one allergen of non-mammalian origin, said device comprising at least one allergen of non-mammalian origin or at least one functional fragment thereof, wherein said at least one allergen of non-mammalian origin or said at least one fragment has a mammalian or mammalian-like glycosylation pattern.

Another aspect of the present invention relates to a kit for screening patients sensitized to at least one allergen comprising
a) at least one allergen of non-mammalian origin or at least one functional fragment thereof, wherein said at least one allergen of non-mammalian origin or said at least one fragment has a mammalian or mammalian-like glycosylation pattern, and
b) at least one further allergen of non-mammalian origin or at least one fragment thereof comprising an amino acid sequence substantially identical to the at least one allergen of non-mammalian origin or at least functional fragment thereof, wherein the at least one further allergen of non-mammalian origin or the at least one fragment thereof has a glycosylation pattern naturally occurring on said at least one further allergen.

### BRIEF DESCRIPTION OF THE FIGURES

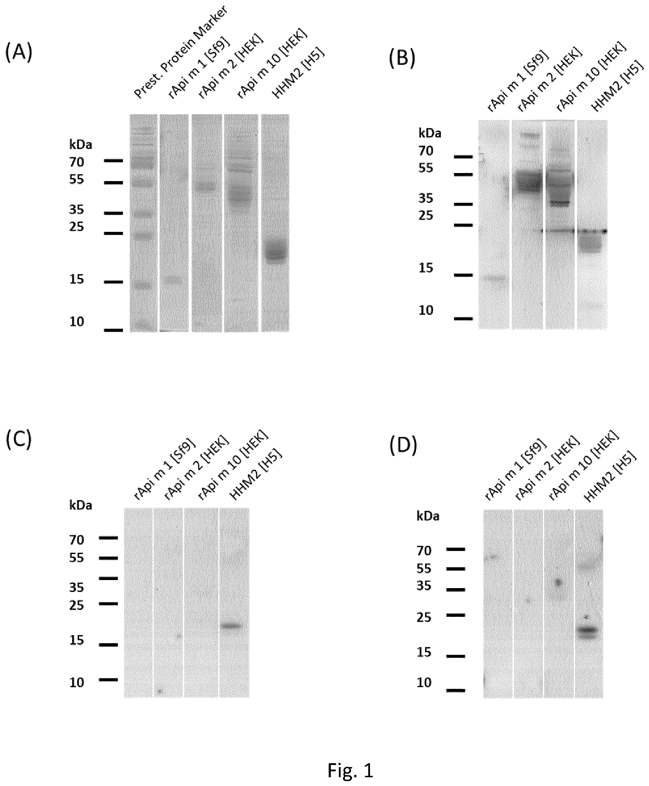

Fig. 1 shows the purification and CCD-specific IgE reactivity of recombinantly expressed bee venom allergens and a CCD marker. **(A)** Coomassie-blue stained SDS-PAGE of purified recombinant insect cell expressed Api m 1 in which the glycosylation site has been removed by mutation (Gattinger, P et al. (2021). J Allergy Clin Immunol, 147(4), 1502-1504.e5.) and HHM2 and HEK-cell expressed Api m 2, Api m 10. Nitrocellulose-blotted purified rApi m 1, rApi m 2, rApi m 10 and HHM 2 detected with **(B)** anti-His-tag antibodies or serum IgE from **(C)** patient #1 or **(D)** patient #2 containing carbohydrate-specific IgE. Molecular weights are indicated in kDa.

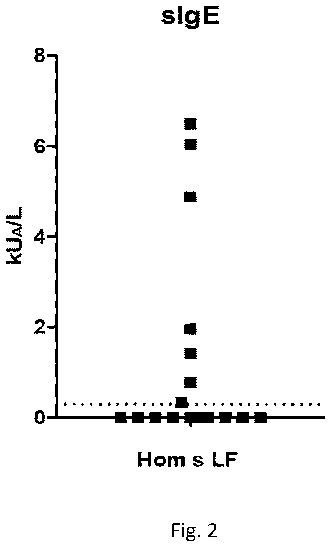

Fig. 2 shows measurement of IgE-specific for a CCD marker after addition of a CCD-blocker. Serum samples from allergic patients (n=17) where measured with ALEX² (Allergy Xplorer) which includes a CCD-blocker that is added to the sera. Shown is the specific IgE reactivity (y-axis) in kUA/L to the glycosylated protein Hom s LF (CCD marker) on the array. Dashed line represents 0.3 kUA/L the cut-off value. IgE reactivity to the CCD marker could not be inhibited by the CCD blocker.

### DESCRIPTION OF EMBODIMENTS

"Allergens of non-mammalian origin", as used herein, refers to allergens which are not naturally occurring in mammals. Examples of such allergens include allergens of plant, fungal, and vertebrate origin, meaning that such allergens are naturally occurring in plants, fungi, and vertebrates.

The term "functional fragment", as used herein, refers to fragments of allergens comprising IgE binding sites. Functional fragments of allergens can be identified by using methods known in the art. Such methods may involve IgE molecules known to bind to a respective full-length allergen.

The presence of allergen specific IgE molecules in a sample can be determined by contacting the sample with an allergen or a functional fragment thereof. After contacting the IgE comprising sample with the allergen or a functional fragment thereof it is determined whether the IgE molecules bind to allergen or a functional fragment thereof. This can be done with methods known in the art using, for instance, ELISA(enzyme-linked immunosorbent assay)-based methods.

The sample of an individual to be used in determining the presence of IgE molecules can also be considered as an IgE comprising sample. Preferred samples are blood samples or protein comprising fractions thereof, in particular serum.

Mammalian or mammalian-like glycosylation patterns can be obtained by expressing allergens of non-mammalian origin or the at least one fragment thereof in mammalian cells or in other cells capable to glycosylate proteins and polypeptides with mammalian or mammalian-like glycosylation patterns (see Marth, J et al. Nat Rev Immunol 8(2008) :874-887, in particular Fig. 1).

Allergens or functional fragments thereof having a mammalian or mammalian-like glycosylation pattern do not comprise CCDs (as reviewed in Altmann F. Int Arch Allergy Immunol 142 (2007) :99-115) .

The allergens of the present invention are proteinaceous allergens. A proteinaceous allergen is a protein, a polypeptide or a peptide.

Another aspect of the present invention relates to method for determining whether a sample comprises allergen specific IgE molecules binding to a cross-reactive carbohydrate determinant (CCD) of an allergen of non-mammalian origin or to the proteinaceous part of said allergen, comprising the steps of:
a) providing at least one first allergen of non-mammalian origin or at least one functional fragment thereof, wherein said at least one first allergen of non-mammalian origin or said at least one fragment thereof has a mammalian or mammalian-like glycosylation pattern,
b) providing at least one second allergen of non-mammalian origin or at least one functional fragment thereof comprising an amino acid sequence substantially identical to the at least one first allergen of non-mammalian origin or functional fragment thereof, wherein said at least one second allergen of non-mammalian origin or said at least one fragment thereof has a glycosylation pattern naturally occurring on said allergen,
c) contacting a first part of said sample with the at least one first allergen of non-mammalian origin or functional fragment thereof and a second part of said sample with the at least one second allergen of non-mammalian origin or functional fragment thereof.

In particular in methods for determining whether a sample comprises allergen specific IgE molecules binding to CCDs it is advantageous to contact a part of the sample with one or more positive controls as defined below. The use of such positive control allows the identification of IgE molecules binding directly to CCDs.

Another aspect of the present invention relates to a method for determining a sensitization profile of an individual comprising the steps of:
a) providing a sample of an individual comprising IgE molecules and
b) contacting said sample with a multiplicity of allergens of non-mammalian origin or functional fragments thereof to determine whether IgEs comprised in the sample bind to one or more of said allergens or fragments thereof giving the sensitization profile of said individual, wherein
at least a part of said allergens of non-mammalian origin or functional fragments has a mammalian or mammalian-like glycosylation pattern.

The method for determining a sensitization profile of an individual comprises the step of contacting the sample with a multiplicity of allergens or fragments thereof as defined herein. A "multiplicity" of allergens of non-mammalian origin or fragments thereof includes at least 2, preferably at least 5, more preferably at least 10, more preferably at least 20, different allergens and fragments thereof. In a particular preferred embodiment of the present invention "multiplicity" refers to 2 to 100, preferably 5 to 100, more preferably 10 to 100, more preferably 20 to 100, different allergens of non-mammalian origin and fragments thereof.

According to a preferred embodiment of the present invention the sensitization profile of an individual can be determined once or at different points of time. A one-time sensitization profile allows to determine the status of an individual. A sensitization profile taken at different points of time allows to monitor the sensitization progress or the therapy of an individual.

Furthermore, the sensitization profile of an individual can also be determined by using single allergens or groups of allergens comprising 2 to 50, 2 to 40, 2 to 30, 2 to 20 or 2 to 10 different allergens in the determination of the presence of allergen specific IgE molecules in a sample at different points of time. The results of these different analysis can be combined to give a sensitization profile of an individual.

A further aspect of the present invention relates to a method for diagnosing an allergy in a subject comprising the steps of:
a) providing at least one first allergen of non-mammalian origin or a functional fragment thereof, wherein said at least one first allergen of non-mammalian origin or said fragment thereof has a mammalian or mammalian-like glycosylation pattern,
b) providing at least one second allergen of non-mammalian origin or a functional fragment thereof comprising an amino acid sequence substantially identical to the at least one first allergen of non-mammalian origin or functional fragment thereof, wherein said at least one second allergen of non-mammalian origin or said fragment thereof has a glycosylation pattern naturally occurring on said at least one second allergen of non-mammalian origin,
c) contacting a first part of said sample with the at least one first allergen of non-mammalian origin or functional fragment thereof and a second part of said sample with the at least one second allergen of non-mammalian origin or functional fragment thereof,
d) diagnosing an allergy if binding of IgE molecules in the sample to the at least one first allergen of non-mammalian origin or functional fragment thereof and a reduced binding of IgE molecules in the sample to the at least one second allergen of non-mammalian origin or functional fragment thereof is determined.

"Substantially identical" amino acid sequence, as used herein, means that the amino acid sequences of the at least one first and of the at least one second allergen or fragment thereof are at least 90%, preferably at least 95%, more preferably at least 98%, in particular 100%, identical.

Percent identity between sequences may be determined using one or more computer algorithms or programs known in the prior art or described herein. According to the present invention, the Basic Local Alignment Search Tool (BLAST) provided by the National Center for Biotechnology Information (NCBI) (Altschul et al., 1990), is preferably used. The BLAST software series contains several programs, including a tool referred to as "BLAST 2 Sequences", which is used for direct pairwise comparison of two nucleotide or amino acid sequences. "BLAST 2 Sequences" can also be accessed and used interactively via the NCBI World Wide Web page on the Internet. The blastp program uses as defaults a word length of 3 and an expectation (E) of 0.05 and the BLOSUM62 scoring matrix (Henikoff & Henikoff, 1989), alignments (B) of 50, expectation (E) of 0.05, M = 1, N = -2.

"Glycosylation pattern naturally occurring on said allergen", as used herein, means that the respective allergen carries its natural glycosylation pattern and can, thus, be considered as the wild-type glycosylation pattern. Extracts from naturally occurring sources or allergens isolated from such sources comprise a naturally occurring glycosylation pattern.

The methods of the present invention involve the detection of IgE molecules binding to allergens of non-mammalian origin or fragments thereof in a sample which are known to comprise at least one glycan chain attached thereto. However, the methods of the present invention may also include the detection of IgE molecules binding to allergens of mammalian origin or fragments thereof or of allergens of non-mammalian and mammalian origin which do not comprise glycan chains attached thereto. Such IgE molecules can be detected in a sample if respective allergens or fragments are added to the test setup next to allergens of non-mammalian origin having a mammalian or mammalian-like glycosylation pattern.

According to a preferred embodiment of the present invention said mammalian or mammalian-like glycosylation pattern is a primate, preferably a human, glycosylation pattern.

Allergens of non-mammalian origin or of mammalian origin having a primate, preferably a human, glycosylation pattern are particularly preferred since primate glycan chains, in particular human glycan chains, do not comprise alpha-galactose. Alpha-galactose is known to be responsible for the so-called alpha-galactose allergy or mammalian meat allergy. Alpha-galactose allergy is a reaction to the carbohydrate galactose-alpha-1,3-galactose ("alpha-galactose"). Hence, IgEs of samples from patients having an alpha-galactose allergy may bind to non-human and non-primate mammalian or mammalian-like glycosylation patterns present on the at least one allergen or fragment thereof resulting in a wrong-positive diagnosis of an allergy.

Hence, it is particularly preferred that the mammalian or mammalian-like glycosylation pattern is a primate, preferably a human, glycosylation pattern, when at least one allergen is of non-mammalian or mammalian origin.

According to another preferred embodiment of the present invention the at least one allergen or the at least one fragment have a primate, preferably a human, N-glycosylation pattern and/or a primate, preferably a human, O-glycosylation pattern.

According to a preferred embodiment of the present invention said allergen(s) of non-mammalian origin is (are) a respiratory allergen of non-mammalian origin or a food allergen of non-mammalian origin.

According to a further preferred embodiment of the present invention the respiratory allergen is of plant or animal origin.

Particularly preferred allergens are selected from the following groups of allergens:
*Animalia Arthropoda*
   *Aedes aegypti* (Yellow fever mosquito): Aed a 1, Aed a 2, Aed a 3, Aed a 4, Aed a 5, Aed a 6, Aed a 7, Aed a 8, Aed a 10, Aed a 11, Aed a 12
   *Apis mellifera* (Honey bee): Api m 1, Api m 2, Api m 3, Api m 4, Api m 5, Api m 6, Api m 7, Api m 8, Api m 9, Api m 10, Api m 11, Api m 12
   *Blattella germanica* (German cockroach): Bla g 1, Bla g 2, Bla g 3, Bla g 4, Bla g 5, Bla g 6, Bla g 7, Bla g 8, Bla g 9, Bla g 11, Bla g 12
   *Blomia tropicalis* (Storage mite): Blo t 1, Blo t 2, Blo t 3, Blo t 4, Blo t 5, Blo t 6, Blo t 7, Blo t 8, Blo t 10, Blo t 11, Blo t 12, Blo t 13, Blo t 18, Blo t 19, Blo t 21, Blo t 24, Blo t 26, Blo t 31, Blo t 32, Blo t 41
   *Dermatophagoides farinae* (American house dust mite): Der f 1, Der f 2, Der f 3, Der f 4, Der f 5, Der f 6, Der f 7, Der f 8, Der f 10, Der f 11, Der f 13, Der f 14, Der f 15, Der f 16, Der f 17, Der f 18, Der f 20, Der f 21, Der f 22, Der f 23, Der f 24, Der f 25, Der f 26, Der f 27, Der f 28, Der f 29, Der f 30, Der f 31, Der f 32, Der f 33, Der f 34, Der f 35, Der f 36, Der f 37, Der f 38, Der f 39, Der f 40
   *Dermatophagoides pteronyssinus* (European house dust mite): Der p 1, Der p 2, Der p 3, Der p 4, Der p 5, Der p 6, Der p 7, Der p 8, Der p 9, Der p 10, Der p 11, Der p 13, Der p 14, Der p 15, Der p 16, Der p 18, Der p 20, Der p 21, Der p 23, Der p 24, Der p 25, Der p 26, Der p 27, Der p 28, Der p 29, Der p 30, Der p 31, Der p 32, Der p 33, Der p 36, Der p 37, Der p 38, Der p 39, Der p 40
   *Lepidoglyphus destructor* (Storage mite): Lep d 2, Lep d 5, Lep d 7, Lep d 10, Lep d 13
   *Penaeus monodon* (Black tiger shrimp): Pen m 1, Pen m 2, Pen m 3, Pen m 4, Pen m 6, Pen m 7, Pen m 8, Pen m 13, Pen m 14
   *Periplaneta americana* (American cockroach): Per a 1, Per a 2, Per a 3, Per a 4, Per a 5, Per a 6, Per a 7, Per a 8, Per a 9, Per a 10, Per a 11, Per a 12, Per a 13, Per a 14, Per a 15, Per a 16, Per a 17, Per a 18, Per a 19, Per a 20
   *Solenopsis invicta* (Red imported fire ant): Sol i 1, Sol i 2, Sol i 3, Sol i 4
   *Tyrophagus putrescentiae* (Storage mite): Tyr p 1, Tyr p 2, Tyr p 3, Tyr p 4, Tyr p 7, Tyr p 8, Tyr p 10, Tyr p 11, Tyr p 13, Tyr p 20, Tyr p 28, Tyr p 31, Tyr p 32, Tyr p 34, Tyr p 35, Tyr p 36
   *Vespula vulgaris* (Yellow jacket): Ves v 1, Ves v 2, Ves v 3, Ves v 5, Ves v 6
*Animalia Chordata*
   *Bos domesticus (Bos taurus)* (Domestic cattle): Bos d 2, Bos d 3, Bos d 4, Bos d 5, Bos d 6, Bos d 7, Bos d 8, Bos d 9, Bos d 10, Bos d 11, Bos d 12, Bos d 13
   *Canis familiaris (C. lupus familiaris)* (Domestic dog): Can f 1, Can f 2, Can f 3, Can f 4, Can f 5, Can f 6, Can f 7, Can f 8
   *Cavia porcellus* (Guinea pig): Cav p 1, Cav p 2, Cav p 3, Cav p 4, Cav p 6
   *Cyprinus carpio* (Common carp): Cyp c 1, Cyp c 2
   *Equus caballus* (Domestic horse): Equ c 1, Equ c 2, Equ c 3, Equ c 4, Equ c 5, Equ c 6
   *Felis domesticus (F. catus)* (Domestic cat): Fel d 1, Fel d 2, Fel d 3, Fel d 4, Fel d 5, Fel d 6, Fel d 7, Fel d 8
   *Gadus callarias* (Baltic cod): Gad c 1
   *Gallus domesticus (G. gallus)* (Chicken): Gal d 1, Gal d 2, Gal d 3, Gal d 4, Gal d 5, Gal d 6, Gal d 7, Gal d 8, Gal d 9, Gal d 10
   *Oryctolagus cuniculus* (Rabbit): Ory c 1, Ory c 2, Ory c 3, Ory c 4
   *Salmo salar* (Atlantic salmon): Sal s 1, Sal s 2, Sal s 3, Sal s 4, Sal s 6, Sal s 7, Sal s 8
*Animalia Nematoda*
   *Anisakis simplex* (Herring worm): Ani s 1, Ani s 2, Ani s 3, Ani s 4, Ani s 5, Ani s 6, Ani s 7, Ani s 8, Ani s 9, Ani s 10, Ani s 11, Ani s 12, Ani s 13, Ani s 14
*Fungi Ascomycota*
   *Alternaria alternate* (*Alternaria* plant rot fungus): Alt a 1, Alt a 3, Alt a 4, Alt a 5, Alt a 6, Alt a 7, Alt a 8, Alt a 10, Alt a 12, Alt a 13, Alt a 14, Alt a 15
   *Aspergillus fumigatus* (Common mold): Asp f 1, Asp f 2, Asp f 3, Asp f 4, Asp f 5, Asp f 6, Asp f 7, Asp f 8, Asp f 9, Asp f 10, Asp f 11, Asp f 12, Asp f 13, Asp f 15, Asp f 16, Asp f 17, Asp f 18, Asp f 19, Asp f 22, Asp f 23, Asp f 24, Asp f 27, Asp f 28, Asp f 29, Asp f 34, Asp f 35, Asp f 36, Asp f 37, Asp f 38, Asp f 39
   *Cladosporium herbarum* (Fungus of plants): Cla h 2, Cla h 5, Cla h 6, Cla h 7, Cla h 8, Cla h 9, Cla h 10, Cla h 12
   *Penicillium citrinum:* Pen c 3, Pen c 13, Pen c 19, Pen c 22, Pen c 24, Pen c 30, Pen c 32
*Fungi Basidiomycota*
   *Malassezia sympodialis* (Skin-colonizing yeast): Mala s 1, Mala s 5, Mala s 6, Mala s 7, Mala s 8, Mala s 9, Mala s 10, Mala s 11, Mala s 12, Mala s 13
*Plantae basal Mesangiosperms*
   *Macadamia integrifolia* (Macadamia): Mac i 1, Mac i 2
   *Papaver somniferum* (Opium poppy): Pap s 1, Pap s 2, Pap s 3
   *Platanus acerifolia* (London plane tree): Pla a 1, Pla a 2, Pla a 3, Pla a 4, Pla a 5, Pla a 6, Pla a 7
*Plantae Eudiokotyledons*
   *Actinidia deliciosa* (Green kiwi fruit): Act d 1, Act d 2, Act d 3, Act d 4, Act d 5, Act d 6, Act d 7,
   Act d 8, Act d 9, Act d 10, Act d 11, Act d 12, Act d 13
   *Alnus glutinosa* (Alder): Aln g 1, Aln g 4
   *Ambrosia artemisiifolia* (Short ragweed): Amb a 1, Amb a 2, Amb a 3, Amb a 4, Amb a 5, Amb a 6, Amb a 7, Amb a 8, Amb a 9, Amb a 10, Amb a 11, Amb a 12
   *Anacardium occidentale* (Cashew): Ana o 1, Ana o 2, Ana o 3
   *Apium graveolens* (Celery): Api g 1, Api g 2, Api g 3, Api g 4, Api g 5, Api g 6, Api g 7
   *Arachis hypogaea* (Peanut, groundnut): Ara h 1, Ara h 2, Ara h 3, Ara h 4, Ara h 5, Ara h 6, Ara h7 , Ara h 8, Ara h 9, Ara h 10, Ara h 11, Ara h 12, Ara h 13, Ara h 14, Ara h 15, Ara h 16, Ara h 17, Ara h 18
   *Artemisia annua* (Sweet Wormwood): Art an 1, Art an 2, Art an 3, Art an 4, Art an 7, Art an 14
   *Artemisia vulgaris* (Mugwort, wormwood): Art v 1, Art v 2, Art v 3, Art v 4, Art v 5, Art v 6
   *Bertholletia excelsa* (Brazil nut): Ber e 1, Ber e 2
   *Betula verrucosa (Betula pendula)* (European white birch): Bet v 1, Bet v 2, Bet v 3, Bet v 4, Bet v 6, Bet v 7, Bet v 8
   *Chenopodium album* (Lamb's quarters, white goosefoot): Che a 1, Che a 2, Che a 3
   *Citrus sinensis* (Sweet orange): Cit s 1, Cit s 2, Cit s 3, Cit s 7
   *Corylus avellana* (Hazelnut): Cor a 1, Cor a 2, Cor a 6, Cor a 8, Cor a 9, Cor a 10, Cor a 11, Cor a 12, Cor a 13, Cor a 14, Cor a 15, Cor a 16
   *Daucus carota* (Carrot): Dau c 1, Dau c 4, Dau c 5
   *Fagopyrum esculentum* (Common buckwheat): Fag e 2, Fag e 3, Fag e 4, Fag e 5
   *Fraxinus excelsior* (European ash): Fra e 1
   *Glycine max* (Soybean): Gly m 1, Gly m 2, Gly m 3, Gly m 4, Gly m 5, Gly m 6, Gly m 7, Gly m 8
   *Helianthus annuus* (Sunflower): Hel a 1, Hel a 2, Hel a 3, Hel a 6, Hel a 15, Hel a 16, Hel a 17
   *Hevea brasiliensis* (Para rubber tree (latex)): Hev b 1, Hev b 2, Hev b 3, Hev b 4, Hev b 5, Hev b 6, Hev b 7, Hev b 8, Hev b 9, Hev b 10, Hev b 11, Hev b 12, Hev b 13, Hev b 14, Hev b 15
   *Juglans regia* (English walnut): Jug r 1, Jug r 2, Jug r 3, Jug r 4, Jug r 5, Jug r 6, Jug r 7, Jug r 8
   *Kali turgidum* (Prickly saltwort [Salsola kali]): Sal k 1, Sal k 2, Sal k 3, Sal k 4, Sal k 5, Sal k 6, Sal k 7
   *Lens culinaris* (Lentil): Len c 1, Len c 2, Len c 3
   *Malus domestica* (Apple): Mal d 1, Mal d 2, Mal d 3, Mal d 4
   *Mangifera indica* (Mango): Man i 1, Man i 2, Man i 4
   *Olea europaea* (Olive): Ole e 1, Ole e 2, Ole e 3, Ole e 4, Ole e 5, Ole e 6, Ole e 7, Ole e 8, Ole e 9, Ole e 10, Ole e 11, Ole e 12, Ole e 13, Ole e 14, Ole e 15
   *Parietaria judaica* (Pellitory of the wall): Par j 1, Par j 2, Par j 3, Par j 4
   *Pistacia vera* (Pistachio): Pis v 1, Pis v 2, Pis v 3, Pis v 4, Pis v 5
   *Pisum sativum* (Pea): Pis s 1, Pis s 2, Pis s 3
   *Plantago lanceolata* (English plantain): Pla l 1, Pla l 2
   *Prunus armeniaca* (Apricot): Pru ar 1, Pru ar 3, Pru ar 5
   *Prunus avium* (Sweet cherry): Pru av 1, Pru av 2, Pru av 3, Pru av 4, Pru av 7
   *Prunus dulcis* (Almond): Pru du 1, Pru du 3, Pru du 4, Pru du 5, Pru du 6, Pru du 8, Pru du 10
   *Prunus persica* (Peach): Pru p 1, Pru p 2, Pru p 3, Pru p 4, Pru p 7, Pru p 9, Pru p 10
   *Pyrus communis* (Pear): Pyr c 1, Pyr c 3, Pyr c 4, Pyr c 5
   *Sesamum indicum* (Sesame): Ses i 1, Ses i 2, Ses i 3, Ses i 4, Ses i 5, Ses i 6, Ses i 7
   *Sinapis alba* (Yellow mustard): Sin a 1, Sin a 2, Sin a 3, Sin a 4
   *Solanum lycopersicum (Lycopersicon esculentum)* (Tomato): Sola l 1, Sola l 2, Sola l 3, Sola l 4,Sola l 5, Sola l 6, Sola l 7
   *Solanum tuberosum* (Potato): Sola t 1, Sola t 2, Sola t 3, Sola t 4
   *Vigna radiata* (Mung bean): Vig r 1, Vig r 2, Vig r 3, Vig r 4, Vig r 5, Vig r 6
   *Vitis vinifera* (Grape): Vit v 1
*Plantae Liliopsida* (monocots)
   *Cynodon dactylon* (Bermuda grass): Cyn d 1, Cyn d 7, Cyn d 12, Cyn d 15, Cyn d 22, Cyn d 23, Cyn d 24
   *Phleum pratense* (Timothy): Phl p 1, Phl p 2, Phl p 3, Phl p 4, Phl p 5, Phl p 6, Phl p 7, Phl p 11, Phl p 12, Phl p 13
   *Triticum aestivum* (Wheat): Tri a 12, Tri a 14, Tri a 15, Tri a 17, Tri a 18, Tri a 19, Tri a 20, Tri a 21, Tri a 25, Tri a 26, Tri a 27, Tri a 28, Tri a 29, Tri a 30, Tri a 31, Tri a 32, Tri a 33, Tri a 34, Tri a 35, Tri a 36, Tri a 37, Tri a 39, Tri a 40, Tri a 41, Tri a 42, Tri a 43, Tri a 44, Tri a 45
*Plantae Pinopsida*
   *Chamaecyparis obtusa* (Japanese cypress): Cha o 1, Cha o 2, Cha o 3
   *Cryptomeria japonica* (Sugi, Japanese cedar): Cry j 1, Cry j 2, Cry j 7
   *Cupressus sempervirens* (Common cypress): Cup s 1, Cup s 2, Cup s 3, Cup s 7
   *Juniperus ashei* (Mountain cedar): Jun a 1, Jun a 2, Jun a 3, Jun a 7

According to a preferred embodiment of the present invention said allergen of non-mammalian origin or said at least one fragment is immobilized on a solid support. Please consider also the possibility that the allergen is in solution!! For example, it may be used to adsorb antibodies and reduce the binding of antibodies to solid-phase bound allergen.

The at least one allergen of non-mammalian origin of the present invention or fragment thereof is preferably immobilized on a solid support to facilitate the detection/determination of IgEs binding to said molecules. The solid support may have varying shapes depending on the method for detecting the interaction between the IgE molecules present in a sample and the allergens of non-mammalian origin or its functional fragments.

According to another preferred embodiment of the present invention a part of the sample is contacted additionally with at least one further allergen of non-mammalian origin or at least one fragment thereof comprising an amino acid sequence substantially identical to the at least one allergen of non-mammalian origin or functional fragment thereof, wherein the at least one further allergen of non-mammalian origin or at least one fragment thereof has a wild-type, i.e. naturally occurring, glycosylation pattern.

According to the present invention one or more positive controls to determine the presence of IgE molecules binding specifically to CCDs can be included in the methods of the present invention. Positive-controls allow to determine the presence of CCD specific IgE molecules which are responsible for false-positive results in the diagnosis of allergies.

The positive controls of the present invention are molecules carrying CCDs. Preferred CCDs include alpha-galactose comprising glycan chains, MUXF3 (obtained e.g. from bromelain), HHM1 and HHM2 (see e.g. Gattinger P et al. EBioMedicine 39(2019):33-43. doi: 10.1016/j.ebiom.2018.12.002). The molecules carrying CCDs are preferably proteins such as bromelain (Ana c 2), horse radish peroxidase (Mari A. Int Arch Allergy Immunol 129(2002): 286-295), lactoferrin or human serum albumin (HSA) (Holzweber F et al. Allergy 68(2013) 1269-1277).

Another aspect of the present invention relates to diagnostic device for screening patients sensitized to at least one allergen of non-mammalian origin, said device comprising at least one allergen of non-mammalian origin or at least one functional fragment thereof, wherein said at least one allergen of non-mammalian origin or said at least one fragment has a mammalian or mammalian-like glycosylation pattern.

A diagnostic device can be any device comprising an allergen. However, diagnostic devices comprising allergens immobilized on a solid support, like diagnostic chips, are particularly preferred.

According to a preferred embodiment of the present invention the device comprises further at least one further allergen of non-mammalian origin or at least one fragment thereof comprising an amino acid sequence substantially identical to the at least one allergen of non-mammalian origin or at least functional fragment thereof, wherein the at least one further allergen of non-mammalian origin or the at least one fragment thereof has a glycosylation pattern naturally occurring on said at least one further allergen.

According to another preferred embodiment of the present invention at least one allergen of non-mammalian origin or at least one functional fragment thereof and the at least one further allergen of non-mammalian origin or at least one fragment thereof is immobilized on a solid support.

A further aspect of the present invention relates to a kit for screening patients sensitized to at least one allergen comprising
a) at least one allergen of non-mammalian origin or at least one functional fragment thereof, wherein said at least one allergen of non-mammalian origin or said at least one fragment has a mammalian or mammalian-like glycosylation pattern, and
b) at least one further allergen of non-mammalian origin or at least one fragment thereof comprising an amino acid sequence substantially identical to the at least one allergen of non-mammalian origin or at least functional fragment thereof, wherein the at least one further allergen of non-mammalian origin or the at least one fragment thereof has a glycosylation pattern naturally occurring on said at least one further allergen.

The kit of the present invention may further comprise one or more positive controls as defined above.

### EXAMPLES

### Example 1

### Methods

### Expression and purification of recombinant allergens

Recombinant not-glycosylated Api m 1 was expressed in Sf9 insect cells and purifies as previously described (Gattinger, P et al. (2021). J Allergy Clin Immunol, 147(4), 1502-1504.e5. https://doi.org/10.1016/j.jaci.2020.10.002). HHM2 was expressed in High-Five insect cells and purified as previously described (Gattinger, P. et al. (2019). EBioMedicine, 39, 33-43) .

For expression of rApi m 2 and rApi m 10 in HEK cells, cDNA encoding for the allergens (Api m 2 Genbank accession Nr.: AAA27730.1 and Api m 10 Genbank accession Nr.: ABF21078.1) without endogenous signal peptides, were codon optimized for the expression in HEK cells. A 5' DNA coding for a N-terminal IL-2 signal peptide (MYRMQLLSCIALSLALVTNS; SEQ ID No. 1) and a 3' DNA coding for a C-terminal hexahistidine tag where added and the synthetic genes were cloned in frame into the mammalian expression vector pcDNA3.1(+) (Genscript, Netherlands). Expression and purification was performed as previously described (Gattinger, P. et al. (2022). Allergy, 77(8), 2431-2445) .

### Immunoblotting

Purified recombinant bee venom allergens and glycosylation marker HHM 2 were subjected to SDS PAGE (12.5% SDS polyacrylamide gels), blotted onto nitrocellulose membranes, probed with an α-histidin-tag mouse IgGi antibody (Dianova, Hamburg, Germany) and bound IgGi was visualized with an alkaline phosphatase labeled anti-mouse IgGi antibody (BD, San Jose, CA, US) as previously described (Gattinger, P. et al. (2019). EBioMedicine, 39, 33-43). To assess the CCD-specific IgE-reactivity of the recombinant allergens the nitrocellulose-blotted proteins were incubated with patients' sera that were 1:10 diluted in gold buffer [50 mM sodium phosphate pH 7.4, 0.5% (v/v) Tween-20, 0.5% (w/v) BSA and 0.05% (w/v) sodium azide]. Bound IgE was detected with 125I -labeled anti-human IgE (BSM Diagnostica, Austria) and visualized by autoradiography. Serum samples of patient#1 and patient #2 used had CCD-specific IgE as previously determined by ELISA and by quantitative ImmunoCAP measurements (Gattinger, P. et al. (2019). EBioMedicine, 39, 33-43. https://doi.org/10.1016/j.ebiom.2018.12.002).

### Results

Purified, Coomassie-stained (Figure 1A) and immunoblotted bee venom allergens and glycosylation marker HHM2 detected with an anti-His-tag antibody (Figure 1B). For non-glycosylated, insect cell expressed rApi m 1 a band at 16kDa was observed (Figure 1A, B). Glycosylated, HEK-cell expressed rApi m 2 and rApi m 10 were detected at 55 kDa and 50-55 kDa, respectively (Figure 1A, B). HHM2 was detected with an approximate molecular weight of 23 kDa.

IgE immunoblot experiments were performed with serum samples from two patients which had shown IgE-reactivity to CCDs in ELISA and quantitative ImmunoCAP experiments. However, neither of the recombinant bee venom allergens rApi m 1, rApi m 2 and rApi m 10 showed specific IgE-reactivity with these serum samples (Figure 1C, D) whereas the glycosylated CCD marker HHM 2 shows IgE reactivity with both serum samples at about 23 kDa (Figure 1C, D).

### Discussion

The recombinant expression of allergens in mammalian cells (e.g., HEK-cells, CHO-cells) can be used to bypass false-positive serological test results in allergy diagnosis. The mammalian cells have the capacity to express folded allergens in high yields. However, due to the different expression of glucosidases and glycosyltransferases than plants and insects, mammalian cell lines do not attach IgE-reactive glycan moieties, also known as classical CCDs, to the recombinantly expressed allergens.

### Example 2: CCD-inhibition in a commercially available test system (ALEX², Allergy Xplorer, MacroArray, Austria) is incomplete

### Methods

Serum samples from 17 allergic patients were analyzed regarding their IgE sensitization with the commercial available multiplex assay ALEX² (Allergy Xplorer) from Macroarray Diagnostics GmbH, Austria, according to manufacturer's instructions. Specific IgE values above 0.3 kUA/L are considered as IgE-positive.

### Results and Discussion

The *in vitro* multiplex allergy test ALEX² has an integrated CCD-blocker to eliminate CCD-reactive antibodies to assure that only specific IgE to a protein epitopes on the allergens included in the test is detected. The test system included several glycoallergens of which several are derived from natural allergen sources. Recombinant human lactoferrin (Hom s LF), which harbors a plant-derived CCD-profile is included in the test to evaluate the degree of blocking IgE reactivity to CCD epitopes. If complete CCD-inhibition is achieved by the CCD-blocker the levels of Hom s LF should be below 0.3 kUA/L. Serum samples from 17 allergic patients (with detailed clinical symptoms ranging from rhino-conjunctivitis, atopic dermatitis and asthma to severe anaphylactic reactions) were tested with the above-mentioned multiplex assay. The procedure was performed according to manufacturer's instructions.

In 410 of analyzed samples (n=7) CCD-sIgE was detected as IgE-reactivity above 0.3 kUA/L to Hom s LF (Fig. 2) after following the manufacturers recommendations. The integrated CCD-blocker of the ALEX² (Allergy Xplorer) was therefore not able to fully block CCD-specific IgE in 41% of tested serum samples as IgE reactivity to the plant expressed CCD-bearing control protein Hom s LF was detectable in these samples.

Gattinger et al. (EBioMedicine. 2019 Jan;39:33-43. doi: 10.1016/j.ebiom.2018.12.002. Epub 2018 Dec 20; Table 2) also showed that a complete inhibition of CCD-specific IgE could not be obtained with another CCD blocker.

## Claims

1. A method for determining the presence of IgE molecules binding to one or more allergens of non-mammalian origin in a sample comprising the step of contacting said sample with at least one allergen of non-mammalian origin or a functional fragment thereof, wherein the at least one allergen or the functional fragment has a mammalian or mammalian-like glycosylation pattern.

2. A method for determining a sensitization profile of an individual comprising the steps of:
a) providing a sample of an individual comprising IgE molecules and
b) contacting said sample with a multiplicity of allergens of non-mammalian origin or functional fragments thereof to determine whether IgEs comprised in the sample bind to one or more of said allergens or fragments thereof giving the sensitization profile of said individual, wherein
at least a part of said allergens of non-mammalian origin or functional fragments has a mammalian or mammalian-like glycosylation pattern.

3. The method of claim 2, wherein the sensitization profile of said individual is determined at different points of time.

4. A method for diagnosing an allergy in a subject comprising the steps of:
a) providing at least one first allergen of non-mammalian origin or a functional fragment thereof, wherein said at least one first allergen of non-mammalian origin or said fragment thereof has a mammalian or mammalian-like glycosylation pattern,
b) providing at least one second allergen of non-mammalian origin or a functional fragment thereof comprising an amino acid sequence substantially identical to the at least one first allergen of non-mammalian origin or functional fragment thereof, wherein said at least one second allergen of non-mammalian origin or said fragment thereof has a glycosylation pattern naturally occurring on said at least one second allergen of non-mammalian origin,
c) contacting a first part of said sample with the at least one first allergen of non-mammalian origin or functional fragment thereof and a second part of said sample with the at least one second allergen of non-mammalian origin or functional fragment thereof,
d) diagnosing an allergy if binding of IgE molecules in the sample to the at least one first allergen of non-mammalian origin or functional fragment thereof and a reduced binding of IgE molecules in the sample to the at least one second allergen of non-mammalian origin or functional fragment thereof is determined.

5. The method according to any one of claims 1 to 4, wherein the mammalian or mammalian or mammalian-like glycosylation pattern is a primate, preferably a human, glycosylation pattern.

6. The method according to any one of claims 1 to 5, wherein the at least one allergen or the at least one fragment have a primate, preferably a human, N-glycosylation pattern and/or a primate, preferably a human, O-glycosylation pattern.

7. The method according to any one of claims 1 to 6, wherein the allergen of non-mammalian origin is a respiratory allergen of non-mammalian origin or a food allergen of non-mammalian origin.

8. The method according to claim 7, wherein the respiratory allergen is of plant or animal origin.

9. The method according to any one of claims 1 to 8, wherein said allergen of non-mammalian origin or said at least one fragment is immobilized on a solid support.

10. The method according to any one of claims 1 to 3 or 5 to 9, wherein a part of the sample is contacted additionally with at least one further allergen of non-mammalian origin or at least one fragment thereof comprising an amino acid sequence substantially identical to the at least one allergen of non-mammalian origin or functional fragment thereof, wherein the at least one further allergen of non-mammalian origin or at least one fragment thereof has a glycosylation pattern naturally occurring on said at least one further allergen.

11. A diagnostic device for screening patients sensitized to at least one allergen of non-mammalian origin, said device comprising at least one allergen of non-mammalian origin or at least one functional fragment thereof, wherein said at least one allergen of non-mammalian origin or said at least one fragment has a mammalian or mammalian-like glycosylation pattern.

12. The diagnostic device according to claim 11, wherein the device comprises further at least one further allergen of non-mammalian origin or at least one fragment thereof comprising an amino acid sequence substantially identical to the at least one allergen of non-mammalian origin or at least functional fragment thereof, wherein the at least one further allergen of non-mammalian origin or the at least one fragment thereof has a glycosylation pattern naturally occurring on said at least one further allergen.

13. The diagnostic device according to claim 11 or 12, wherein at least one allergen of non-mammalian origin or at least one functional fragment thereof and the at least one further allergen of non-mammalian origin or at least one fragment thereof is immobilized on a solid support.

14. Kit for screening patients sensitized to at least one allergen comprising
a) at least one allergen of non-mammalian origin or at least one functional fragment thereof, wherein said at least one allergen of non-mammalian origin or said at least one fragment has a mammalian or mammalian-like glycosylation pattern, and
b) at least one further allergen of non-mammalian origin or at least one fragment thereof comprising an amino acid sequence substantially identical to the at least one allergen of non-mammalian origin or at least functional fragment thereof, wherein the at least one further allergen of non-mammalian origin or the at least one fragment thereof has a glycosylation pattern naturally occurring on said at least one further allergen.
